# EUROPEAN PATENT SPECIFICATION

(11) **EP 2 863 826 B1**
(45) Date of publication and mention of the grant of the patent: **20.03.2019**
(21) Application number: 13810185.2
(22) Date of filing: 21.06.2013
(51) Int. Cl.: A61B 18/20, A61B 8/08, A61B 18/22, A61B 18/18, A61B 17/34

(54) **ELECTROSURGICAL DEVICE INCORPORATING A PHOTO-ACOUSTIC SYSTEM FOR INTERROGATING/IMAGING TISSUE**
ELEKTROCHIRURGISCHE VORRICHTUNG MIT EINEM FOTOAKUSTISCHEN SYSTEM ZUR INTERROGATION/BILDGEBUNG VON BIOLOGISCHEM GEWEBE
DISPOSITIF ÉLECTROCHIRURGICAL COMPRENANT UN SYSTÈME PHOTOACOUSTIQUE POUR INTERROGER/IMAGER UN TISSU

(30) Priority: 26.06.2012 US 201261664535 P; 19.06.2013 US 201313922006
(43) Date of publication of application: 29.04.2015
(73) Proprietor: Covidien LP, Mansfield, MA 02048 (US)
(72) Inventor: NAU, William H. Jr., Longmont, Colorado 80504 (US)
(74) Representative: Soames, Candida Jane
(86) International application number: PCT/US2013/046949
(87) International publication number: WO 2014/004267

(56) References cited:
- EP-A1- 2 316 359
- WO-A2-2009/055705
- WO-A2-2011/053931
- US-A- 5 156 144
- US-A- 6 134 003
- US-A1- 2003 120 268
- US-A1- 2005 010 199
- US-A1- 2007 005 061
- US-A1- 2008 108 867
- US-A1- 2010 082 019
- US-A1- 2011 098 572
- US-A1- 2011 184 322
- US-A1- 2011 282 192
- US-B2- 7 179 254
- US-B2- 8 181 839

## Description

### BACKGROUND

### 1. Technical Field

The present disclosure relates to electrosurgical instruments used during a surgical procedure. More particularly, the present disclosure relates to systems and methods for interrogating and/or imaging tissue using photo-acoustic signals.

### 2. Background of the Related Art

Energy-based tissue treatment is well known in the art. Various types of energy (e.g., electrical, ultrasonic, microwave, cryogenic, thermal, laser, etc.) are applied to tissue to achieve a desired result. Electrosurgical instruments have become widely used by surgeons in recent years. By and large, most electrosurgical instruments are hand-held instruments, e.g., electrosurgical pencils, electrosurgical forceps, endoscopic instruments such as monopolar forceps, bipolar forceps or a combination monopolar/bipolar forceps, ultrasonic hand tools, microwave probes.

After performing a surgical procedure using an electrosurgical instrument, the instrument has to be removed before a user can determine the extent of the treatment and whether additional treatment may be needed. If additional treatment is needed, the electrosurgical instrument would have to be reinserted into the patient which may cause additional concerns.

Surgical clamps comprising ultrasonic receivers and reflectors are disclosed in US2011/0184322 A1.

### SUMMARY

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)". For the purposes of this description, a phrase in the form "at least one of A, B, or C" means "(A), (B), (C), (A and B), (A and C), (B and C), or (A, B and C)".

As shown in the drawings and described throughout the following description, as is traditional when referring to relative positioning on a surgical instrument, the term "proximal" refers to the end of the apparatus that is closer to the user or generator and the term "distal" refers to the end of the apparatus that is farther away from the user or generator. The term "clinician" refers to any medical professional (i.e., doctor, surgeon, nurse, or the like) performing a medical procedure involving the use of aspects of the present disclosure described herein.

Electromagnetic (EM) energy is generally classified by increasing frequency or decreasing wavelength into radio waves, microwaves, infrared, visible light, ultraviolet, X-rays and gamma-rays. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the term "RF" generally refers to electromagnetic waves having a lower frequency than microwaves. As used herein, the term "ultrasound" generally refers to cyclic sound pressure with a frequency greater than the upper limit of human hearing. The terms "tissue" and "lumen" may be used interchangeably since it is believed that the present disclosure may be employed to seal and cut tissue or seal and cut lumens utilizing the same principles described herein.

The phrase "transmission line" generally refers to any transmission medium that can be used for the propagation of signals from one point to another.

The phrase "electrosurgical instrument" may refer to any instrument configured to output electrosurgical energy such as electrosurgical pencils, electrosurgical forceps, endoscopic instruments such as monopolar forceps, bipolar forceps or a combination monopolar/bipolar forceps, ultrasonic hand tools, and microwave probes, antennas, needles, or catheters. The phrase "electrosurgical energy" may refer to energy used to perform a surgical procedure such as electromagnetic energy or acoustic energy. The phrase "end effector" may refer to any device capable of emitting electromagnetic energy or acoustic energy to treat tissue within the vicinity of the end effector. Types of end effectors may include antennas, ultrasonic transducers, electrodes, jaw members, probes, acoustic waveguides, combinations thereof or the like.

The term "generator" may refer to a device capable of providing electrosurgical energy. Such device may include a power source and electrical components (analog and/or digital components) capable of modifying the energy outputted by the power source to output energy having a desired energy modality. The phrase "energy modality" may refer to the characteristics of the outputted electrosurgical energy. Such characteristics may include an energy mode, which includes the type of energy (e.g., RF, microwave, ultrasound, etc.) and/or waveform (e.g., sine wave, square wave, triangle wave, sawtooth wave, composite waves, etc.), and/or energy level.

The electrosurgical instruments herein may utilize one or more sensors configured to detect one or more properties of tissue and/or the ambient environment. Such properties include, but are not limited to: tissue impedance, tissue type, tissue clarity, tissue compliance, temperature of the tissue or jaw members, water content in tissue, jaw opening angle, water motility in tissue, energy delivery, and jaw closure pressure.

The electrosurgical instruments used herein may also utilize a controller to receive various inputs from a number of switches and or sensors and control the energy modality of the energy outputted by the electrosurgical instrument, The controller may include any type of computing device, computational circuit, or any type of processor or processing circuit capable of executing a series of instructions that are stored in a memory. The controller may include multiple processors and/or multicore central processing units (CPUs) and may include any type of processor, such as a microprocessor, digital signal processor, microcontroller, or the like.

Any of the herein described methods, programs, algorithms or codes may be converted to a programming language or computer program. A "Programming Language" and "Computer Program" is any-language used to specify instructions to a computer, and includes (but is not limited to) these languages and their derivatives: Assembler, Basic, Batch files, BCPL, C, C+, C++, Delphi, Fortran, lava, JavaScript, Machine code, operating system command languages, Pascal, Pearl, PU1, scripting languages, Visual Basic, metalanguages which themselves specify programs, and all first, second, third, fourth, and fifth generation computer languages. Also included are database and other data schemas, and any other meta-languages. For the purposes of this definition, no distinction is made between languages which are interpreted, compiled, or use both compiled and interpreted approaches. For the purposes of this definition, no distinction is made between compiled and source versions of a program. Thus reference to a program, where the programming language could exist in more than one state (such as source, compiled, object, or linked) is a reference to any and all states. The definition also encompasses the actual instructions and the intent of those instructions.

Any of the herein described methods, programs, algorithms or codes may be contained on one or more machine-readable media or memory. The term "memory" may include a mechanism that provides (i.e., stores and/or transmits) information in a form readable by a machine such a processor, computer, or a digital processing device. For example, a memory may include a read only memory (ROM), random access memory (RAM), magnetic disk storage media, optical storage media, flash memory devices, or any other volatile or non-volatile memory storage device. Code or instructions contained thereon can be represented by carrier wave signals, infrared signals, digital signals, and by other like signals.

In an embodiment of the present disclosure, an electrosurgical system is provided that includes a first energy source configured to output a first energy and an electrosurgical instrument. The electrosurgical instrument includes a transmission line coupled to the energy source and configured to emit the first energy. A mirror reflects the first energy towards a target tissue and an ultrasonic transducer receives a second energy from the target tissue. The second energy is converted into an electrical signal that is sent to a controller to convert the electrical signal into an image.

The electrosurgical instrument may also include a motor coupled to the mirror and configured to translate and/or rotate the mirror along a longitudinal axis defined through the electrosurgical instrument.

The energy source may be a laser or a microwave generator and the transmission line may be an optical fiber or an antenna. In the embodiments described herein, the electrosurgical instrument may be an ablation needle or a catheter.

In another embodiment of the present disclosure, an electrosurgical instrument is provided that includes a shaft, a first jaw member and a second jaw member both disposed at a distal end of the shaft and that grasps tissue therebetween. The electrosurgical instrument also includes an energy source configured to emit a first energy directed to a target tissue and an ultrasonic transducer configured to receive a second energy from the target tissue and convert the second energy into an electrical signal.

The energy source may be in the first jaw member while the ultrasonic transducer may be in the second jaw member. The energy source may be movable from a first position to a second position along a longitudinal axis defined in the first jaw member.

The electrosurgical instrument may also include a mirror configured to reflect the first energy from the energy source toward the target tissue. The mirror and/or ultrasonic transducer may be in the first jaw member.

The electrosurgical instrument may also include a motor configured to move the mirror relative to a longitudinal axis to translate or rotate the mirror along the longitudinal axis defined through the shaft.

In yet another embodiment of the present disclosure, the ultrasonic transducer, the mirror, and the motor may be disposed in the shaft.

In another embodiment of the present disclosure, an electrosurgical instrument is provided that includes a housing and a transmission line adapted to connect to a first energy source and configured to emit a first energy. A mirror reflects the first energy towards a target tissue and an ultrasonic transducer receives a second energy from the target tissue. The second energy is converted into an electrical signal that is sent to a controller to convert the electrical signal into an image. The first energy emitted by the transmission line is different than the second energy received by the ultrasonic transducer.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a system block diagram of an electrosurgical system according to an embodiment of the present disclosure;
Fig. 2 is a system block diagram of a photo acoustic detection system according to an embodiment of the present disclosure;
Figs. 3A and 3B are schematic diagrams of an electrosurgical instrument according to embodiments of the present disclosure;
Fig. 4 is a schematic diagram of an electrosurgical instrument according to another embodiment of the present disclosure;
Fig. 5 is a schematic diagram of an electrosurgical instrument according to yet another embodiment of the present disclosure;
Fig. 6 is a schematic diagram of an electrosurgical instrument according to yet another embodiment of the present disclosure; and
Fig. 7 is a schematic diagram of an electrosurgical instrument according to yet another embodiment of the present disclosure.

### DETAILED DESCRIPTION

Particular embodiments of the present disclosure are described hereinbelow with reference to the accompanying drawings; however, the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures.

The present disclosure is directed to electrosurgical instruments that incorporate a photo-acoustic tomography (PAT) system to image and/or interrogate tissue. PAT uses pulses of light or microwave (MW) energy to generate an acoustic wave that can be used to image tissue or measure and/or monitor physiological events in real time. PAT provides a measurement of optical contrast with an acoustic signal to overcome the diffusion limit associated with optics when interrogating tissue. In PAT, an acoustic pressure (P) is proportional to a temperature rise (ΔT) which is proportional to an optical absorption coefficient (µₐ) times an optical fluence rate (F). A temperature rise of 1 degree (due to absorption of optical or MW energy) generates an acoustic pressure wave of 8 mbar. The PAT system of the present disclosure can be used to image, monitor, or interrogate tissue on scales as small as the organelle level to as large as the organ level. Incorporating a PAT system into an electrosurgical device would provide, among other things, surface or subsurface imaging capabilities to determine seal quality, histological diagnosis or identification of surface or subsurface tissue structures in real time, or real-time subsurface imaging capabilities to determine the extent of the coagulative necrosis zone during a thermal ablation procedure.

Further, by incorporating a PAT system in an electrosurgical device, real-time interrogation of lymph nodes and suspicious masses for cancer detection prior to energy delivery may be accomplished. Differentiating between cancerous and benign tumors, or determining if the margins of the ablation zone are positive or negative for a residual tumor may also be performed.

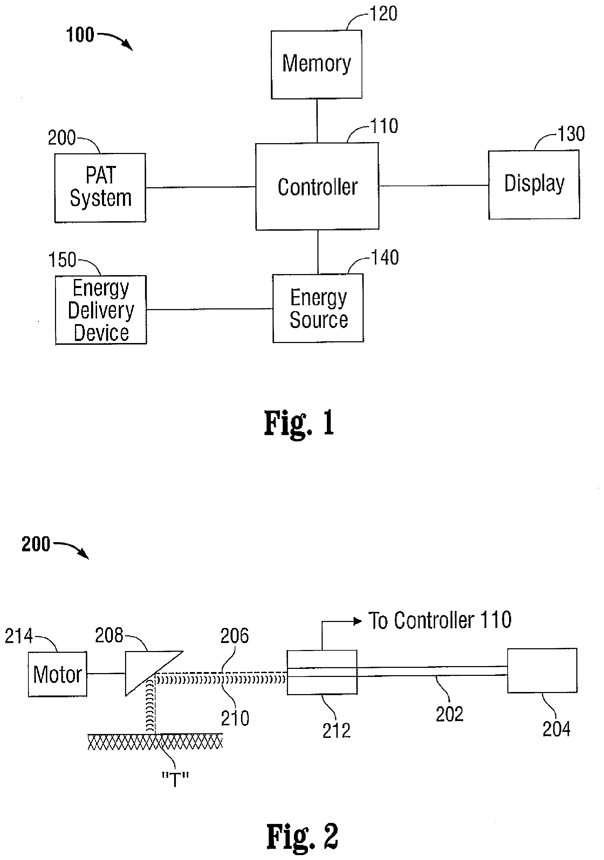

Turning to Fig. 1, a system block diagram of an electrosurgical system is shown generally as 100. System 100 includes an energy source 140 configured to output electrosurgical energy to an energy delivery device 150. Energy source 140 may supply MW energy and/or RF energy. Energy delivery device 150 may be an antenna or electrodes that are incorporated into a probe, needle, catheter, or forceps. Energy delivery device 150 outputs the electrosurgical energy to tissue within the vicinity of energy delivery device 150. System 100 also includes a controller 110 that is configured to receive various inputs and outputs a control signal to energy source 140 to control the output of energy source 140. Controller 110 may receive inputs from an activation button that controls application of the electrosurgical energy and/or sensors. Controller 110 is coupled to a memory 120 that has programs and/or algorithms used to control the output of the electrosurgical instrument 100 (e.g., open loop or closed loop feedback programs). Memory 120 may also store a number of programs for controlling a PAT system 200. Memory 120 may be a separate component or integrated into controller 110. Controller 110 also receives a signal from a PAT system 200 and controls display 130 to display the imaged and/or interrogated tissue based on the signal from PAT system 200.

Turning to Fig. 2, the PAT system according to an embodiment of the present disclosure is shown generally as 200. PAT system 200 includes a transmission line 202 that transfers energy (represented by dashed line 206) from energy source 204 to tissue "T" by reflecting the energy off of mirror 208. Energy source 204 may be a laser source or MW generator. Energy source 204 may be incorporated into energy source 140 or energy source 204 may be a separate component. Transmission line 202 may be an optical fiber if energy source 204 is a laser or it may be an antenna if energy source 204 is a MW generator.

Energy 206 is reflected off of mirror 208 toward tissue "T". As tissue "T" absorbs energy, the temperature of tissue "T" rises thereby creating an acoustic pressure wave 210. The acoustic pressure wave 210 is reflected off of mirror 208 toward an ultrasonic transducer 212. Ultrasonic transducer 212 converts the received acoustic pressure wave to an electrical signal that is transmitted to controller 110 to be processed. Controller 110 then displays a representation of the tissue "T" on display 130 based on the received electrical signal.

A micro-motor 214 is connected to mirror 208 to move mirror 208 along a longitudinal axis of an electrosurgical instrument, which will be described below, or motor 214 may rotate mirror relative to the longitudinal axis. Motor 214 may be directly coupled to mirror 208 or may be indirectly coupled to mirror 208 via a number of gears (not shown). Motor 214 may be controlled by a switch or any other actuator (not shown) or motor 214 may be controlled by controller 110 according to an algorithm stored in memory 120.

Controller 110 controls PAT system 200 in order to image and/or interrogate tissue "T". When a user wants to image and/or interrogate tissue "T", the user inputs a signal to controller 110 to initiate the imaging /interrogating procedure via a switch or any other actuator (not shown). Controller 110 then controls energy source 204 to emit energy through transmission line 202 to image and/or interrogate tissue "T". Imaging/interrogating may be performed according to an algorithm stored in memory 120 that controls the duration and wavelength of the emitted energy, location of mirror 208, and rotation of mirror 208.

By scanning energy source 204 and/or ultrasonic transducer 212, a two-dimensional (2-D) image of tissue "T" may be obtained. In another embodiment, multiple wavelengths may be used to generate three-dimensional (3-D) images of tissue "T". Additionally, multiple wavelengths may be used to monitor multiple features or events that include, but are not limited to, tissue temperature, tissue oxygen saturation, metabolic rate or oxygen uptake, hematocrit, perfusion rate, etc. The usage of multiple wavelengths to monitor multiple features is based on the optical absorption in the photoacoustic excitation phase. By using multiwavelength measurements, concentrations of multiple chromophores of different colors, such as oxygenated and deoxygenated hemoglobin molecules in red blood cells, can be quantified. Such quantification of hemoglobin can provide functional imaging of the concentration and oxygen saturation of hemoglobin, which are related to hallmarks of cancer. For instance, the concentration of hemoglobin correlates with angiogenesis, whereas the oxygen saturation of hemoglobin correlates with hypermetabolism. Such parameters of hemoglobin can also be used to image brain activity. In addition, extrinsic optical absorption contrast agents can be used to provide molecular imaging of biomarkers.

As an example, energy source 204 may be a laser that emits light having a wavelength between 100 and 1000 nm. The light travels through transmission line 202 toward tissue "T" by reflecting the light off of mirror 208. Tissue "T" absorbs the light causing a rise in temperature of tissue "T". The rise in temperature creates an acoustic wave 210 that is detected by ultrasonic transducer 212. Ultrasonic transducer 212 converts the received acoustic pressure wave to an electrical signal that is transmitted to controller 110, which then displays a representation of the tissue "T" on display 130 based on the received electrical signal.

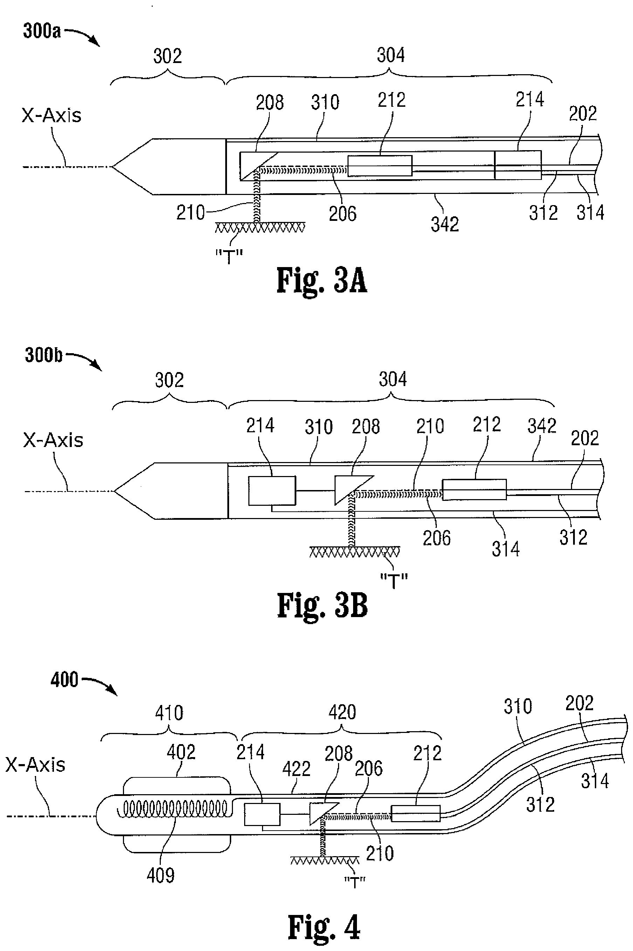

Figs. 3A and 3B depict ablation needles that incorporate a PAT system in accordance with embodiments of the present disclosure. As shown in Figs 3A and 3B, ablation needles 300a and 300b include a distal radiating section 302 that provides treatment to tissue in the vicinity of distal radiating section 302. Distal radiating section 302 is connected to a MW energy source (not shown) via a transmission line 310. Ablation needles 300a and 300b also include a PAT system 304. PAT system 304 includes a transmission line 202 that emits energy 206 toward mirror 208. Mirror 208 reflects energy 206 toward tissue "T" and an acoustic pressure wave 210 created in tissue "T" is reflected by mirror 208 toward ultrasonic transducer 212. Ultrasonic transducer 212 converts acoustic pressure wave 210 into an electrical signal and transmits the electrical signal to a controller (not shown) via transmission line 312.

Motor 214 is connected to mirror 208 to translate mirror 208 proximally or distally along the x-axis. Motor 214 also rotates mirror 208 around the x-axis to obtain a 360 degree field of view. Motor 214 may be placed proximal to the ultrasonic transducer 212 as shown in Fig. 3A or motor 214 may be disposed distal to the mirror 208 as shown in Fig. 3B. Motor 214 is electrically coupled to a controller (not shown in this embodiment) or a switch (not shown) to control translation and/or rotation of mirror 208 via transmission line 314.

PAT system 304 may be incorporated into a shaft 342 of ablation needles 300a or 300b. Shaft 342 may be composed of any material suitable for the transmission of light, MW energy, and/or acoustic pressure waves to and/or from tissue. For instance, materials that may be used to compose shaft 342 include, but are not limited to, polyethylene terephthalate (PET), silicone, polymethylpentene, etc.

Fig. 4 depicts an ablation catheter 400 that incorporates a PAT system in accordance with another embodiment of the present disclosure. Ablation catheter 400 may range in size from 3 French to 30+ French depending on the target lumen. Ablation catheter 400 includes distal radiating section 410 that includes a balloon 402 and an antenna 408. Balloon 402 may be used to center the ablation catheter 400 in a lumen. Ablation catheter also includes a PAT section 420. PAT section 420 includes a transmission line 202 that delivers energy 206 to mirror 208 which, in turn, reflects the energy toward tissue "T". Tissue "T" emits an acoustic pressure wave 210 that is detected by ultrasonic transducer 212 that converts the acoustic pressure wave into an electrical signal that is sent to a controller (not shown in this embodiment) via transmission line 312. A motor 214 is electrically coupled to a controller (not shown) or a switch (not shown in this embodiment) to control translation and/or rotation of mirror 208 via transmission line 314. Shaft 422 of PAT section 420 may be composed of any material suitable for the transmission of light, MW energy, and/or acoustic pressure waves to and/or from tissue.

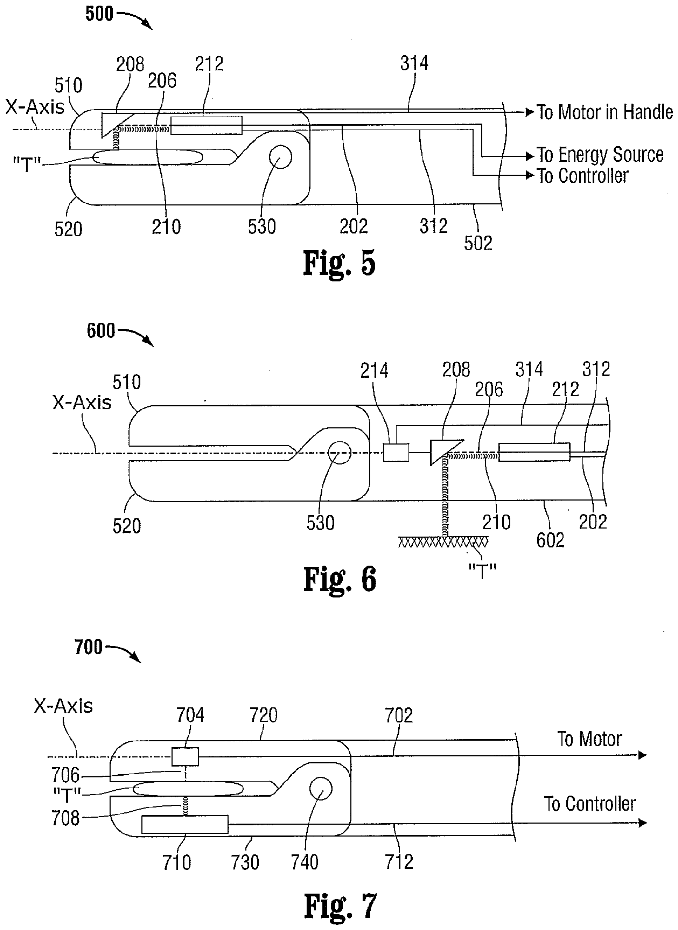

Figs. 5-7 depict various embodiments of monopolar or bipolar forceps incorporating a PAT system in accordance with embodiments of the present disclosure. Turning to Fig. 5, an electrosurgical instrument 500 includes a pair of opposing jaw members 510 and 520 at the distal end of shaft 502 that move relative to each other around a pivot point 530. Jaw member 510 includes a mirror 208 that is connected to a motor (not shown in this figure) in the handle via transmission line 314. The motor translates mirror 208 along the x-axis. Mirror 208 reflects energy 206 from transmission line 202, which is connected to an energy source (not shown in this figure), toward tissue "T" grasped between jaw members 510 and 520. An acoustic pressure wave 210 is emitted from tissue "T" to ultrasonic transducer 212 that converts the acoustic pressure wave to an electrical signal to be transmitted over transmission line 312.

Fig. 6 depicts a distal end of electrosurgical instrument 600 according to another embodiment of the present disclosure. In electrosurgical instrument 600, the PAT system is similar to the PAT system 200 shown in Fig. 2 and is disposed in the shaft 602. Shaft 602 may be composed of any material suitable for the transmission of light, MW energy, and/or acoustic pressure waves to and/or from tissue.

Fig. 7 depicts an electrosurgical instrument 700 in accordance with yet another embodiment of the present disclosure. Electrosurgical instrument 700 includes a pair of opposing jaw members 720 and 730 that move relative to each other around a pivot point 530. Jaw member 720 includes an energy source 704 that emits optical or MW energy 706 toward tissue "T". Energy source 704 is connected to a motor (not shown in this embodiment) that translates energy source along the x-axis. As tissue "T" heats up, an acoustic pressure wave 708 is generated and detected by transducer 710. Transducer 710 converts the acoustic pressure wave 708 into an electrical signal that is sent to a controller (not shown in this embodiment).

The various embodiments disclosed herein may also be configured to work with robotic surgical systems and what is commonly referred to as "Telesurgery". Such systems employ various robotic elements to assist the surgeon in the operating theater and allow remote operation (or partial remote operation) of the electrosurgical instruments described herein. Various robotic arms, gears, cams, pulleys, electric and mechanical motors, etc. may be employed for this purpose and may be designed with a robotic surgical system to assist the surgeon during the course of an operation or treatment. Such robotic systems may include, remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients. The one or more consoles may incorporate a display to display the imaged and/or interrogated tissue based on the signal from the PAT systems described herein.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. The claims can encompass embodiments in hardware, software, or a combination thereof. Those skilled in the art will envision other modifications within the scope of the claims appended hereto.

## Claims

1. An electrosurgical instrument (500), comprising:
a shaft (502);
a first jaw member (510) and a second jaw member (520) disposed at a distal end of the shaft and configured to grasp tissue therebetween;
an energy source (204) configured to emit a first energy directed to target tissue;
an ultrasonic transducer (212) configured to receive a second energy from the target tissue and convert the second energy into an electrical signal;
a mirror (208) configured to reflect the first energy from the energy source toward the target tissue; and
a motor configured to move the mirror relative to a longitudinal axis defined through the shaft,
wherein the mirror is in the first jaw member, or in the shaft.

2. The electrosurgical instrument according to claim 1, wherein the ultrasonic transducer is in the first jaw member and the mirror is in the first jaw member.

3. The electrosurgical instrument according to claim 1, wherein the motor is configured to translate the mirror along the longitudinal axis defined through the shaft.

4. The electrosurgical instrument according to claim 1, wherein the motor is configured to rotate the mirror around the longitudinal axis defined through the shaft.

5. The electrosurgical instrument according to claim 1, wherein the ultrasonic transducer, the mirror, and the motor are disposed in the shaft.

6. The electrosurgical instrument of any preceding claim, being monopolar or bipolar forceps.

7. The electrosurgical instrument of any preceding claim, wherein the first energy is for imaging or measurement and comprises multiple wavelengths.

8. A system comprising the electrosurgical instrument of any preceding claim and an electrosurgical energy source (140).

9. The system of claim 8, wherein the electrosurgical energy source is configured to generate radiofrequency or microwave energy.

10. An electrosurgical system, comprising:
a first energy source configured to output a first energy;
the electrosurgical instrument of claim 1, including:
a housing; and
a transmission line coupled to the first energy source and configured to emit the first energy.

## Patentansprüche

1. Elektrochirurgisches Instrument (500), umfassend:
einen Schaft (502);
ein erstes Backenelement (510) und ein zweites Backenelement (520), die an einem distalen Ende des Schafts angeordnet sind und konfiguriert sind, Gewebe zwischen ihnen zu erfassen;
eine Energiequelle (204), die konfiguriert ist, eine erste Energie abzugeben, die auf Zielgewebe gerichtet ist;
einen Ultraschallwandler (212), der konfiguriert ist, eine zweite Energie vom Zielgewebe zu empfangen und die zweite Energie in ein elektrisches Signal umzuwandeln;
einen Spiegel (208), der konfiguriert ist, die erste Energie von der Energiequelle auf das Zielgewebe hin zu reflektieren; und
einen Motor, der konfiguriert ist, den Spiegel relativ zu einer Längsachse zu bewegen, die durch den Schaft hindurch definiert ist,
wobei sich der Spiegel in dem ersten Backenelement oder in dem Schaft befindet.

2. Elektrochirurgisches Instrument nach Anspruch 1, wobei sich der Ultraschallwandler in dem ersten Backenelement befindet und der Spiegel sich in dem ersten Backenelement befindet.

3. Elektrochirurgisches Instrument nach Anspruch 1, wobei der Motor dafür konfiguriert ist, den Spiegel entlang der Längsachse, die durch den Schaft hindurch definiert ist, zu verschieben.

4. Elektrochirurgisches Instrument nach Anspruch 1, wobei der Motor dafür konfiguriert ist, den Spiegel um die durch den Schaft hindurch definierte Längsachse zu drehen.

5. Elektrochirurgisches Instrument nach Anspruch 1, wobei der Ultraschallwandler, der Spiegel und der Motor im Schaft angeordnet sind.

6. Elektrochirurgisches Instrument nach einem der vorstehenden Ansprüche, dass eine monopolare oder bipolare Zange ist.

7. Elektrochirurgisches Instrument nach einem der vorstehenden Ansprüche, wobei die erste Energie der Bildgebung oder Messung dient und mehrere Wellenlängen umfasst.

8. System, das das elektrochirurgische Instrument nach einem der vorstehenden Ansprüche und eine elektrochirurgische Energiequelle (140) umfasst.

9. System nach Anspruch 8, wobei die elektrochirurgische Energiequelle konfiguriert ist, Radiofrequenz- oder Mikrowellenenergie zu erzeugen.

10. Elektrochirurgisches System, umfassend:
eine erste Energiequelle, die konfiguriert ist, eine erste Energie abzugeben;
das elektrochirurgische Instrument nach Anspruch 1, einschließlich eines Gehäuses; und
eine Übertragungsleitung, die mit der ersten Energiequelle verbunden ist und zur Abgabe der ersten Energie konfiguriert ist.

## Revendications

1. Instrument électrochirurgical (500), comprenant :
un arbre (502) ;
un premier élément de mors (510) et un second élément de mors (520) disposés à une extrémité distale de l'arbre et configurés pour saisir un tissu entre eux ;
une source d'énergie (204) configurée pour émettre une première énergie dirigée sur le tissu cible ;
un transducteur ultrasonique (212) configuré pour recevoir une seconde énergie du tissu cible et convertir la seconde énergie en un signal électrique ;
un miroir (208) configuré pour réfléchir la première énergie de la source d'énergie vers le tissu cible ; et
un moteur configuré pour déplacer le miroir par rapport à un axe longitudinal défini à travers l'arbre,
dans lequel le miroir se trouve dans le premier élément de mors ou dans l'arbre.

2. Instrument électrochirurgical selon la revendication 1, dans lequel le transducteur ultrasonique se trouve dans le premier élément de mors et le miroir se trouve dans le premier élément de mors.

3. Instrument électrochirurgical selon la revendication 1, dans laquelle le moteur est configuré pour effectuer une translation du miroir le long d'un axe longitudinal défini à travers l'arbre.

4. Instrument électrochirurgical selon la revendication 1, dans lequel le moteur est configuré pour faire tourner le miroir autour de l'axe longitudinal défini à travers l'arbre.

5. Instrument électrochirurgical selon la revendication 1, dans lequel le transducteur ultrasonique, le miroir et le moteur sont disposés dans l'arbre.

6. Instrument électrochirurgical selon l'une quelconque des revendications précédentes, qui est un forceps monopolaire ou bipolaire.

7. Instrument électrochirurgical selon l'une quelconque des revendications précédentes, dans lequel la première énergie est destinée à l'imagerie ou à la mesure et comprend de multiples longueurs d'ondes.

8. Système comprenant l'instrument électrochirurgical selon l'une quelconque des revendications précédentes et une source d'énergie électrochirurgicale (140).

9. Système selon la revendication 8, dans lequel la source d'énergie électrochirurgicale est configurée pour générer de l'énergie de radiofréquence ou de l'énergie de microonde.

10. Système électrochirurgical comprenant :
une première source d'énergie configurée pour délivrer une première énergie ;
l'instrument électrochirurgical de la revendication 1 comportant :
un boîtier ;
une ligne de transmission couplée à la première source d'énergie et configurée pour émettre la première énergie.
